(19) Europäisches Patentamt
European Patent Office
Office européen des brevets

(11) **EP 2 210 904 B1**

(12) **EUROPEAN PATENT SPECIFICATION**

(45) Date of publication and mention
of the grant of the patent:
**30.07.2014 Bulletin 2014/31**

(51) Int Cl.:
*C07K 19/00* (2006.01)    *C12P 21/02* (2006.01)
*A61K 38/16* (2006.01)    *A61P 35/00* (2006.01)
*C12N 15/62* (2006.01)

(21) Application number: **08851459.1**

(22) Date of filing: **08.10.2008**

(86) International application number:
**PCT/CN2008/001675**

(87) International publication number:
**WO 2009/065292 (28.05.2009 Gazette 2009/22)**

(54) **METHOD OF PREPARING A FUSION PROTEIN COMPRISING TUMOR NECROSIS FACTOR RELATED APOPTOSIS INDUCING LIGAND AND INTEGRIN LIGAND AND USE THEREOF**

VERFAHREN ZUR HERSTELLUNG EINES FUSIONSPROTEINS MIT TUMORNEKROSEFAKTORBEDINGTE APOPTOSE INDUZIERENDEM LIGAND UND INTEGRIN-LIGAND SOWIE SEINE VERWENDUNG

PROCÉDÉ POUR LA PRÉPARATION D'UNE PROTÉINE DE FUSION COMPRENANT UN LIGAND INDUCTEUR D'APOPTOSE LIÉ AU FACTEUR DE NÉCROSE TUMORALE ET UN LIGAND D'INTÉGRINE ET SON UTILISATION

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **23.10.2007 CN 200710133862**

(43) Date of publication of application:
**28.07.2010 Bulletin 2010/30**

(73) Proprietor: **Nanjing University**
**Jiangsu 210093 (CN)**

(72) Inventors:
• **HUA, Zichun**
**Jiangsu 210093 (CN)**
• **CAO, Lin**
**Weigang, Nanjing 210095 (CN)**

(74) Representative: **Kramer - Barske - Schmidtchen**
**Landsberger Strasse 300**
**80687 München (DE)**

(56) References cited:
**WO-A2-02/061105**    **WO-A2-2007/011217**
**CN-A- 1 419 565**    **CN-A- 1 614 025**
**CN-A- 101 157 729**    **US-B1- 6 521 228**

• **JACOB DIETMAR ET AL: "Suppression of pancreatic tumor growth in the liver by systemic administration of the TRAIL gene driven by the hTERT promoter", CANCER GENE THERAPY, vol. 12, no. 2, February 2005 (2005-02), pages 109-115, XP002609766, ISSN: 0929-1903**
• **ZHAO, L. H ET AL.: 'Cloning, expression and purification ofRGD-sTRAIL and its anti-tumor effects in vitro.' LETTERS IN BIOTECHNOLOGY. vol. 15, no. 3, May 2004, pages 226 - 230, XP009138369**
• **CAO, L. ET AL.: 'Enhancement of antitumor properties of TRAIL by targeted delivery to the tumor neovasculature.' MOL CANCER THER. vol. 7, no. 4, April 2008, pages 851 - 861, XP009138364**
• **TARRUS, M. ET AL.: 'RGD-avidin-biotin pretargeting to integrin enhances the proapoptotic activity of TNF related apoptosis inducing ligand (TRAIL)' APOPTOSIS vol. 13, no. 2, February 2008, pages 225 - 235, XP019569371**

EP 2 210 904 B1

## Description

## FIELD OF THE INVENTION

This present invention relates to the biotechnological field.

## BACKGROUND OF THE INVENTION

Tumor necrosis factor-related apoptosis-inducing ligand (TRAIL) is a family member of tumor necrosis factor superfamily. Similar to other members of the tumor necrosis factor superfamily, the soluble form of tumor necrosis factor-related ligands are bound with the tripolymer-based receptor molecules on the target cell surface in a form of trimer and thus playing the biological functions. The apoptosis inducing function of TRAIL is realized by its interaction with the death receptor 4 (DR4) and DR5 on the tumor cells through death signal transmission. Although other members of tumor necrosis factor superfamily are restricted due to its systemic side effect, the TRAIL is a kind of anti-tumor substance with relative safety and anti-tumor selection. The TRAIL can induce the apoptosis of a variety of tumor cells and cancer cells in vitro, and play better anti-tumor activity in the mice transplanted xenogeneic tumors, including colon cancer, breast cancer, multiple myeloma, glioma, prostate cancer and many other models. Particularly important, when drugs were administered in the body of mice and non-human primates, TRAIL presented less or no toxicity. For the above reasons, the recombinant TRAIL has been used in the clinical trial study for tumor treatment.

However, some recent reports showed that, in addition to inducing tumor cell apoptosis, the TRAIL is also involved in innate immunity and adaptive immunity, associated with autoimmune diseases. For example, recent studies have reported that, the TRAIL can regulate the negative selection or apoptosis of thymus cells during the thymus development process, and plays an important role in inducing autoimmune diseases such as type I diabetes. In addition, the receptors of TRAIL are extensively expressed in the whole body, and the TRAIL also involves in liver cell death and hepatitis. Therefore, repeated and systemic use of high-dose exogenous TRAIL protein may cause unpredictable immunization effect in clinic. For these reports, scientists are very worried about the potential side effect of TRAIL in the repeated and systemic use in clinic. How to avoid the side effect of TRAIL on other tissues for tumor treatment is a challenge in the potential clinic use of TRAIL.

US 6,521,228 discloses a fusion protein comprising a TRAIL polypeptide and a tumor-targeting moiety.

L. H. Zhao et al., Letters in Biotechnology, 2004, vol. 15, no. 3, 226-230, disclose a study on cloning, expression and purification of RGD-sTRAIL and its antitumor effects in vitro, wherein RGD-sTRAIL is a fusion protein.

D. Jacob et al., Cancer Gene Therapy, 2005, vol. 12, no. 2, 109-115, disclose a study on suppression of pancreatic tumor growth in the liver by systemic administration of the TRAIL gene driven by the hTERT promoter.

## SUMMARY OF THE INVENTION

The object of the present invention is to provide an improved method for preparing a TRAIL variant.

The above object is achieved by the method according to claim 1.

Although the expression products of TRAIL in E. coli mainly are the inclusion body products without biological activity, in the present invention, the molecular structure and molecular weight of the TRAIL variant are more complicated than the wild-type of TRAIL, therefore, when expression in E. coli, its inclusion body formation will be more and the purification process will be more complicated. According to the present method, the low temperature cultivation and inducing expression of TRAIL are adopted, which effectively avoid the formation of the inclusion body; meanwhile, by using the biological functions of chelation of metal ions of

TRAIL, metal affinity chromatography and ion-exchange chromatography, the TRAIL and its variants have been effectively purified and high purity of protein products have been obtained; and the purified products have high proportion of polymer form, therefore, they present very good biological property.

There are extremely low expressions of integrins of $\alpha V\beta 3$ and $\alpha V\beta 5$ in the dormant cells and normal vascular cells; however, in many tumor cells (such as melanoma, colon cancer, breast cancer and uterine cancer) and tumor endothelial cells, their expression significantly enhance. The TRAIL variants comprising integrin ligands of $\alpha V\beta 3$ and $\alpha V\beta 5$, TRAIL can significantly enhance the distribution of TRAIL in the tumor tissues and realize the targeting distribution of TRAIL in the tumor tissues, and greatly enhance the anti-tumor effect of TRAIL, meanwhile, significantly reduce the application amount of TRAIL.

Comparing with presently available TRAIL and its variants, the present method provides a TRAIL variant having the following distinct characters:

(1) Better targeting of tumor tissues: the relative selection of the existing TRAIL on the tumor tissues is mainly realized by the death receptors (DR) 4 and DR5 expressed in the tumor tissues, however, the TRAIL variant, in addition to use of DR4 and DR5 expressed in the tumor tissues, also make use of the features of rich expression of integrins of $\alpha V\beta 3$ and $\alpha V\beta 5$ in the tumor tissues, and thus effectively realize the targeting delivery of TRAIL in the tumor tissues.

(2) More efficient anti-tumor effect: because of targeting delivery of TRAIL in the tumor tissues, the TRAIL variant, compared with the same dose of TRAIL, presents better anti-tumor effect no matter used individually or combined use with the existing chemotherapy, radiotherapy, TCM treatment, biological treatment and other methods.

(3) Lower application dose. Because the TRAIL variant has better anti-tumor effect compared with the same dose of TRAIL, when used, it can significantly reduce the amount of TRAIL variant protein under the conditions of same anti-tumor curative effect. The decreased administration dose of TRAIL variant protein will be able to overcome the potential side effect of TRAIL in the treatment of tumors, and at the same time, reduce the application prospect of this strategy, reduce the curative expenses of tumor patients, and achieve the effect of high efficiency, low side effect and low-cost of tumor treatment.

(4) Convenient for expression and production: differing from the tumor-cell specific antibody and antibody-fragment targeting TRAIL fusion protein, the fusion of the TRAIL and the short-peptide ligand of integrins of $\alpha V\beta 3$ and $\alpha V\beta 5$ increases the molecular weight a little, and thus conducive to the gene cloning, expression and production of TRAIL variant, and realize higher yields.

(5) Soluble expression and simple separation and purification: Although the TRAIL can form non-biological activity of inclusion body product when expression in E. coli, however, the molecular structure and molecular weight of TRAIL variants are more complicated than the wild-type of TRAIL, therefore, when expression in E. coli, its inclusion body forms are more serious and its purification are more complicated. According to the claimed method, the low temperature cultivation and inducing expression of TRAIL are adopted, which effectively avoid the formation of the inclusion body; meanwhile, by using the biological functions of chelation of metal ions of TRAIL, metal affinity chromatography and ion-exchange chromatography, the TRAIL and its variants have been effectively purified and high purity of protein products have been obtained; and the purified products have high proportion of polymer form, therefore, they present very good biological property.

## DESCRIPTION OF DRAWINGS

FIG. 1 The purified recombinant human TRAIL and its variant

1A. SDS-PAGE analysis results: 1. molecular weight standard; 2: Human TRAIL; 3. Human TRAIL variant;

1B. Non-reducing and non-denaturing PAGE analysis results: 1: Human TRAIL; 2. Human TRAIL variant.

FIG. 2 Test of promotion of human foreskin microvascular endothelial cell adhesion and well-adhesion capability by human TRAIL variant (Zoom 200)

2A. Human foreskin microvascular endothelial cell adhesion and adhensive to wells coated with human TRAIL variant at 6.4 $\mu$g/ml per well;

2B. Human foreskin microvascular endothelial cell adhesion and adhensive to wells coated with human TRAIL at 6.4 $\mu$g/ml per well.

FIG. 3 Analysis of the in vivo binding of green fluorescein-labeled human TRAIL or its variant with tumor tissue in tumor animal models with a flow cytometer.

Tumor animal models: BALB/c nu/nu nude mice inoculated with COLO-205 tumor cells. Tail vein injection of human TRAIL variant RGD-L-TRAIL-FITC (black ash line, hollow peak chart), human TRAIL-FITC (black line, hollow peak chart), control protein BSA-FITC (light gray line, hollow peak chart) into tumor-bearing mice; after 30 min later, the tumor tissues are peeled off to prepare cell suspension for analysis with a flow cytometer. The negative controls (black solid peak chart) are selected from the tumor animals without injection of any proteins.

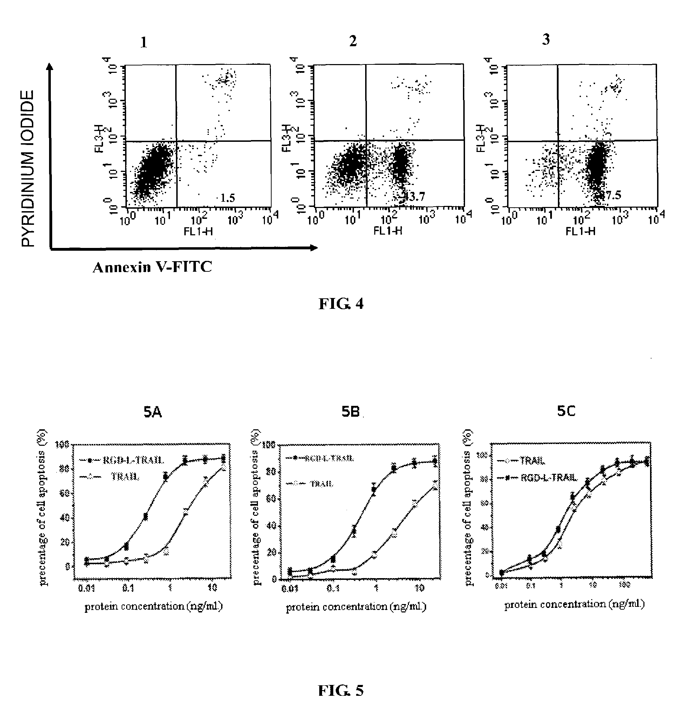

FIG. 4 Test of the capacity of inducing COLO-205 apoptosis of human TRAIL and its variant (2.43 ng/ml) with Annexin V and pyridine iodide (PI) double staining method.

1: control; 2. human TRAIL; 3. human TRAIL variant.

FIG. 5. Analysis on the dose-effect relationship of human TRAIL and its variant induced tumor cell apoptosis.

5A: COLO-205 cell; 5B: Jurkat cell; 5C: MDA-MB-231 cell.

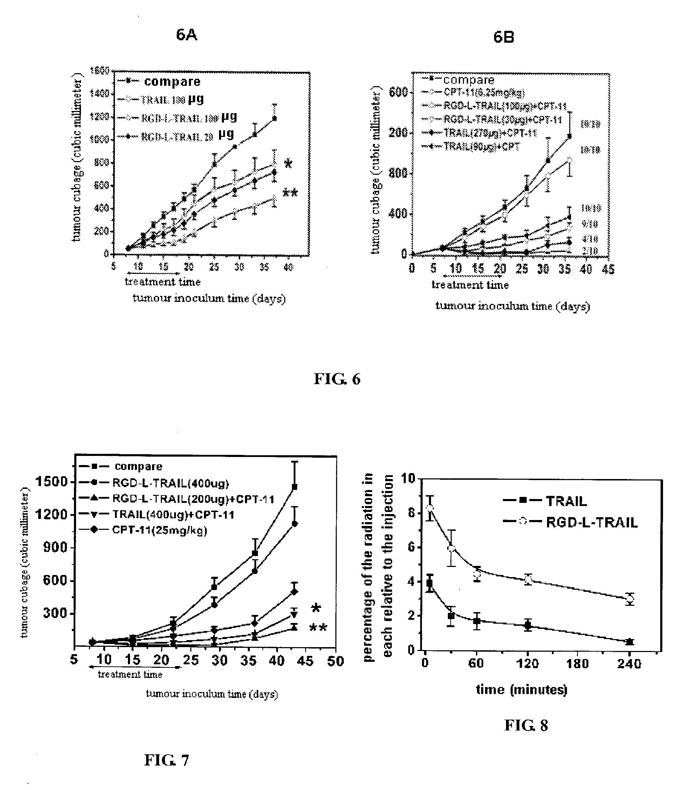

FIG. 6 Individual treatment of human TRAIL and its variant in the COLO-205 tumor model and anti-tumor effect combined with CPT-11.

6A: Individual treatment of human TRAIL and its variants; 6B: CPT-11 combined treatment with human TRAIL and its variant. The data analysis results are represented with the average; the variance is the standard error; * indicates p <0.05; ** indicates p <0.01.

FIG. 7 Individual treatment of human TRAIL and its variant in the HT-29 colon tumor models and anti-tumor effect combined with CPT-11.

Statistical analysis results are represented by average value; the variance is the standard error; * indicates p <0.05; ** indicates p <0.01.

FIG. 8 Analysis of the targeting accumulation effect of human TRAIL and its variant in the tumor tissues of COLO-205 tumor animal models.

Separate injection from tail veins of 100 $\mu$l containing 5 Ci of $^{125}$I-labeled human TRAIL or its variant protein into the COLO-205 tumor bearing nude mice. 5, 30, 60, 120 and 240 minutes after injection, the tumor tissues were peeled off separately and weighed, and the radiation amount of the tumor tissues were measured with a liquid scintillation counter. The amount of radiation quantity in the tumor tissues is represented by the percentage of measuring radiation quantity per gram of tissues and the injected radiation quantity (% ID/g). The results are the average value of three independent tests.

DETAILED DESCRIPTION OF THE INVENTION

Example

(1) Gene cloning, expression and purification of TRAIL variant

The wild-type human TRAIL genes are obtained from reverse transcription of messenger RNA available from human placenta. The PCR primer of TRAIL variant gene fused with ACDCRGDCFC short peptide is: Primer 1:

5'-TGCAGATCATATGGCATGCGACTGCCGT-GGTGA CTGCTT

CTGCGGTGGTGGTGGTGGTGTGAGAGAAA-GAGGTCCTCAG-3' ;

Primer 2 : 5'-ATGGATCCTTAGCCAACTAAAAAGGCCC-3'.

The RGD-L-TRAIL gene of connecting peptide coding of short peptide ACDCRGDCFC and five glycines was obtained through PCR reaction. RGD-L-TRAIL gene was cloned to Novagen's pET-23a expression vector. The resulting recombinant expression plasmids were expressed in the Escherichia coli BL21 (DE3). To obtain soluble expression of RGD-L-TRAIL, the expression condition should be as follows: The overnight growth of recombinant expression bacteria were diluted for 100 times in the LB medium, and cultivated for 2.5 h at 37 °C, and then cultivated for 1-2 h at 24 °C. Under the condition of 24 °C, IPTG was added to 0.6 mM, and then the bacteria were induced for expression at 24 °C overnight. After centrifugation, the bacteria were suspended in lysis buffer (50 mM sodium phosphate, 0.5 M NaCl, 1 mM dithiothreitol, pH 7.6) and lysed through ultrasonication. After separation and purification of RGD-L-TRAIL protein through Ni-NTA agarose gel medium (Qiagen Company), 60 mM imidazole elution peak was collected, and then purified through SP-Sepharose cation resin. In the test, all the water used are ultra-pure water of endotoxin-free. The protein quantitative measurement was carried out with the BCA protein test kit provided by Nanjing Jiancheng Bio-engineering Institute. The purity and molecular weight of TRAIL and it variant protein should be analyzed with silver-staining SDS-PAGE method, and the molecular weight was determined by mass-spectrometry with Applied Biosystems 4700 Proteomics Analyzer.

(2) Cell adhesion assay of human foreskin microvascular endothelial cell

The TRAIL and its variant protein are coated in 96-well PVC plate overnight at 4 °C. The coated 96-well plate was rinsed with 0.9% physiological saline, and then blocked for lh at 1640 culture medium containing 2% bovine serum albumin, and then rinsed again. After the human foreskin microvascular endothelial cells were digested, washed for three times with phosphate buffer and re-suspended into the complete RPMI1640 medium, then added to 96-well plate coated with TRAIL and its variant (40,000 cell per well). After cells were incubated for 1.5 hours in the incubator containing 5% carbon dioxide, washed for three times with physiological saline to remove the non-adherent cells. The adherent cells were fixed for 30 min with phosphate buffer solution (pH 7.3) containing 4% formalin and 2% sucrose, and then stained with 0.5% crystal violet. The fixed stained cells were rinsed for several times, then dissolved with 100 ml DMSO to test the absorbance values at 540 nm. All the tests should be carried out for at least three times independently.

(3) Binding assay of TRAIL and its variant with endothelial cell

The TRAIL and its variant were labeled with fluorescein (Sigma company) respectively, and the labeled proteins were removed of free fluorescein through a Sephadex-G25 size exclusion column. After human foreskin microvascular endothelial cells were digested by trypsin, then washed twice with ice-cold phosphate buffer containing 2% fetal calf serum, and then re-suspended, added with 1 $\mu$g of labeled protein for incubation for 1 h at 4 °C. The fluorescent-labeled bovine serum albumin was used as a control of the test. After washed for three times, analyze the binding capacity of the stained cells with a flow cytometer (Becton Dickinson Company).

(4) Analysis of integrin $\alpha$V$\beta$3 and $\alpha$V$\beta$5 expression abundance.

The cell surface integrin expression abundance was tested with a flow cytometer through indirect labeling method. After digestion, the cells were rinsed twice with ice-cold phosphate buffer containing 2% fetal calf serum, after re-suspended of cells, coated with the following monoclonal antibodies for lh on the ices-anti-human $\alpha$V$\beta$3 integrin antibody MAB 23C6 (eBioscience, Inc.), or anti-human $\alpha$V$\beta$5 integrin antibody MAB 1961 (Chemicon International Inc.). The negative control adopted purified same-isotype of mouse immunoglobulin G (eBioscience Corporation). The cells coated with first antibody were washed twice, then added with green fluorescein conjugated goat anti-mouse immunoglobulin Gl (y) (Caltag Laboratories Inc.), and the secondary antibody was reacted for 30 min by avoiding light, and then washed for three times, then fixed with phosphate buffer containing 4% formalin, and finally the integrin expression abundance was tested on the flow cytometer. All the staining tests should be repeated for three times.

(5) Test of cell apoptosis with Annexin V and pyridine iodide (PI) double staining method

After the cells treated with TRAIL and its variant were digested with trypsin, fetched out from the cultivation plate, then washed twice with phosphate buffer solution, centrifuged for 5 min at 300g centrifugal force. The supernatants were removed, then the cells were re-suspended in 100 μl of binding buffer solution, added with final concentration of 2 μg/ml Annexin V-FITC (BD Pharmgen) for incubation at room temperature. After 10 min, 400 μl of binding buffer was supplemented; the cells were transferred to a flow analysis tube. 1 μg of iodide-pyridine (Sigma Corporation) was added to each tube. The cells were analyzed with a flow cytometer within 30 min. At least three times of tests should be carried out for each cell line.

(6) Caspase-8 and Caspase-3 Activity Assay

Caspase-8 and Caspase-3 activity were determined by fluorescence detection kit (Oncogene Inc.). The test methods followed the methods provided by the factory. The fluorescence values were tested with a microplate reader, and the fluorescence parameters: excitation wavelength: 400 nm, emission wavelength: 505 nm.

(7) Test of anti-tumor efficacy on tumor animal models

5-6 week-old female nude mice were purchased from Shanghai Experimental Animal Center. The nude mice were injected through tail veins with 100 μg of specific antibody, i.e., purified Asialo GM-1 antibody (Wako Chemicals Inc., Japan) that blocks natural killer cells. After 24 hours, the upper right sides of the back of nude mice were inoculated subcutaneously with 100,000 COLO-205 or HT-29 colon cancer cells. When the tumor reached 70 mm$^3$, random grouping and began to treatment. The recombinant TRAIL and its variant proteins were administered intraperitoneally, once daily for 10-14 days. The water-soluble camptothecin CPT-11 (11-hydroxy-camptothecin; Product Name: Irinotecan Hydrochloride Injection; Pharmacia/Upjohn Company's product) was administered intravenously once a week, two times in total. The recombinant proteins and camptothecin were diluted with a phosphate buffer. The tumor size was measured with a caliper, and the calculation formula was:

$$(\text{length} \times \text{width})^2/2.$$

(8) Drug distribution test of recombinant proteins in tumor tissues

The recombinant TRAIL and the TRAIL variant protein were labeled with a radioisotope of iodine 125 labeling kit (Pierce Corp.). The labeling results: the specific activity of $^{125}$I-TRAIL was 7.86 μCi/μg protein, and the specific activity of $^{125}$I-TRAIL variant was 7.49 μCi/μg protein. After the nude mice were inoculated with COLO-205 colon cancer, and when the tumor size reached 400-500 mm$^3$, randomly grouped them into wild-type and variant group. Each group included five time points: 5 min, 30 min, 60 min, 120 min and 240 min, and each time point included three animals. Each tumor-bearing nude mouse was injected with 100 μl of liquid containing 5 μl of protein. At each time point, the mice were sacrificed, and dissected the tumor by surgery, and measured the radiation dose through a liquid scintillation counter. The distribution of the tumor tissue should be represented by the radiation percentage containing in each gram of tissues (relative to the original injection of radioactive counting percentage) (%ID/g).

(9) In vivo binding capability test of recombinant proteins in the body and tumor tissues

The TRAIL and its variant, bovine serum albumin were labeled with green fluorescein. The labeled protein was removed of free fluorescein with a gel filtration of Sephadex-G25. When the tumor grew to 400-500 mm$^3$, injected 500 μg of fluorescent-labeled protein through tail veins of the tumor-bearing mice; after 30 min, killed the mice to remove the tumor, prepare single-cell suspensions of tumor cells, washed with normal saline for several times. Took 60000 cells for test through a cell flow cytometer and analyzed the binding capability of the recombinant proteins on the tumor cell surfaces.

(10) Statistical Analysis

The statistical data analysis was carried out with social scientific data software. All tests should be repeated for three times. The cell apoptosis and adhesion test results were represented with average standard deviation. The tumor size was represented by the standard error. If the P value was less than 0.05, it was regarded significant difference, and less than 0.01, regarded extremely significant difference, indicated by * and ** respectively.

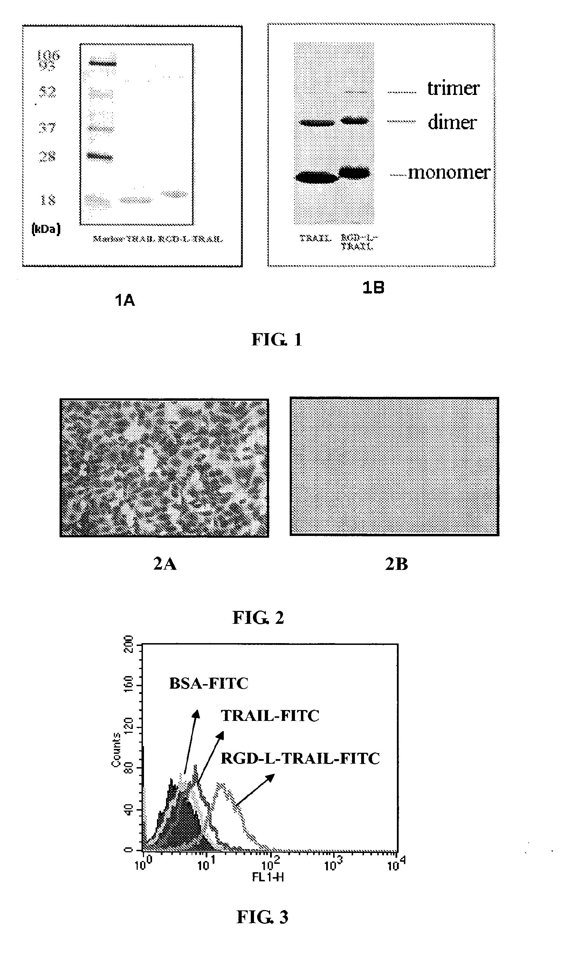

In previous reports, when the wild-type TRAIL was expressed in E. coli, it would exist in the inactive inclusion body; in this invention, the TRAIL variant was added with 4 cysteines (i.e. added with two pairs of disulfide bonds). When the variant was expressed in E. coli, it would more easily form the inclusion body product without biological activity than wild-type TRAIL and the purification became more difficult. In the present invention, by optimizing the expression conditions and separation and purification process, successfully realized soluble form of expression of TRAIL and its variant; while the high purity of TRAIL and its variant proteins with full biological activity obtained through nickel metal affinity column and cation resin purification have a yield of 60 mg/L. The expression products can be demonstrated under 15% denaturation and reducing conditions through silver-stained polyacrylamide gel electrophoresis analysis and mass spectrometry sequencing analysis (Fig. 1A). Also, under the expression conditions and purification process, the TRAIL and its variants have very high proportion of polymer forms, which were very rare in the similar work of TRAIL expression and purification.

The tumor necrosis factor super-family proteins have the features of formation of monomer, dimer and trimer properties, and their biological activities depend on the dimer and trimer forms, without exception for the TRAIL. To test whether after fusion of ACDCRGDCFC domain, it has impact on the formation of TRAIL and variant polymer, a non-denaturing non-reducing polyacrylamide gel electrophoresis analysis was carried out, which showed that the recombinant TRAIL and its variant proteins have the ability to form polymers. The results showed that there were three bands of molecular weight of about 20,000, 40,000 and 60,000 daltons for both TRAIL and its variant protein, which respectively were monomer, dimer and trimer (Fig. 1B). More important, the proportions of monomers, dimers and trimers of TRAIL and its variant proteins were basically the same. This demonstrated that the expressed and purified TRAIL and its variant proteins indeed have correct spatial conformation structure, and have higher biological activity than TRAIL expressed in E. coli reported previously.

ACDCRGDCFC is the ligand of αVβ3 and αVβ5 integrins containing RGD sequences with the structure of ring cake or kringle-like that can form two pairs of disulfide bonds, which have better affinity and selectivity for integrins αVβ3 and αVβ5 than simple RGD sequences. In the present invention, the results showed that: human foreskin microvascular endothelial cells do not adhere to the culture plate wells coated with TRAIL, but the TRAIL variant with fusion of αVβ3 and αVβ5 integrin ligands could make the human foreskin microvascular endothelial cell adhesive to the culture plate wells coated with TRAIL variant protein and in a dose-dependent manner (Fig. 2). This indicated that the short peptide ligand of integrin αVβ3 and αVβ5 among TRAIL variant endowed TRAIL with an ability of adhesion to endothelial cell and cell's adhesion to culture wells.

Free RGD-L-TRAIL, RGD-L-TRAIL (C230G) (a kind of TRAIL variant fused with αVβ3 and αVβ5 integrin ligands together with a mutation at TRAIL's activity center making TRAIL losing its biological activity) and TRAIL protein were added to the culture plates coated with TRAIL variant protein for competitive test. The test results showed that, RGD-L-TRAIL or RGD-L-TRAIL (C230G) protein could significantly reduce the quantity of human foreskin microvascular endothelial cell adhesion to the microplates coated with RGD-L-TRAIL, but TRAIL has no such ability.

In the present invention, the integrin αVβ3 and αVβ5 receptors are designed target. We firstly conducted analysis on the expression of integrins αVβ3 and αVβ5 on the endothelial cells and tumor cells surface. The analysis results through a flow cytometer showed that the expression level of integrins αVβ3 and αVβ5 is maximum on human foreskin microvascular endothelial cell surfaces; in addition, it should be noted that, a variety of tumor cells have different degree of expression of integrins αVβ3 and αVβ5, of which, moderate abundance of integrins αVβ3 and αVβ5 expression in on T lymphoma cells; relatively weak integrin αVβ3 expression in colon cancer cell COLO-205, but the level of integrin αVβ5 was relatively higher; there were no expression of integrin αVβ3 and lower expression level of integrin αVβ5 in the breast cancer cell MDA-231.

We also further evaluated the capacity of fluorescein-labeled TRAIL and its variant directly binding with human foreskin microvascular endothelial cells. The results of flow cytometry showed that the TRAIL variant's binding capacity with human foreskin microvascular endothelial cells is much higher than that of the TRAIL. These results revealed that: the Integrin αVβ3 and αVβ5 ligand short peptide can significantly enhance the specific binding capacity of TRAIL variant for the vascular endothelial cells.

We also evaluated the binding condition of TRAIL and its variant to tumor cells in the tumor-bearing nude mice. The fluorescein-labeled TRAIL and its variant, and negative control of bovine serum albumin were injected to tumor-bearing mice inoculated with COLO-205 tumor cells. 30 min later, the mice were sacrificed and the tumors were removed, the capacity of tumor cells binding with fluorescein-labeled TRAIL and its variants was analyzed (Fig. 3). The results showed that the TRAIL variant has stronger binding capacity for the tumor cells than that of the wild-type TRAIL. These results confirmed that the integrins αVβ3 and αVβ5 short peptide ligand endowed the selective binding property of the TRAIL to tumor tissues and enhance its tumor-targeting ability.

We used COLO-205, Jurkat, and MDA-MB-231 tumor cells to evaluate the activity of TRAIL variant to induce tumor cell apoptosis. The tumor cells, after a series of concentration gradients of TRAIL variant or TRAIL-induced treatment, were analyzed by the Annexin V-FITC and PI double staining method with a flow cytometer. The results showed that, compared with TRAIL, in the COLO-205 colon cancer cells and Jurkat T lymphoma cells, TRAIL variants have stronger apoptosis-inducing ability; however, in the breast cancer cells MDA-MB-231, the ability of TRAIL variant to induce cell apoptosis only was slightly stronger than that of wild-type TRAIL. For example, for COLO-205 cells, 2.43 ng/ml TRAIL-induced apoptosis rate was 43.7%; while TRAIL variant with the same dose could achieve the apoptosis rate as high as 87.5 % (Fig. 4). The medial lethal doses of TRAIL on COLO-205, Jurkat and MDA-MB-231 were 3.5, 6.7 and 2.3 ng/ml respectively; while the corresponding medial lethal doses of TRAIL variant reduced to 0.37, 0.41 and 1.2 ng/ml; for COLO-205, Jurkat, and MDA-MB-231 cells, the dose of TRAIL variant was reduced by 9.5, 16.34, 1.9 times respectively (Fig. 5). TRAIL variant has a better ability to induce apoptosis of tumor cells.

For the $\alpha V\beta5$ for $\alpha V\beta3$ integrin expression on tumor cells, the TRAIL variant can targetedly deliver to the tumor cell surface and tumor blood vessel cell surfaces. In the present invention, TRAIL variant could significantly enhance the tumor-targeting cell apoptosis. In COLO-205 and Jurkat tumor cells, the TRAIL variant enhanced 8-10 times of tumor cell-inducing apoptosis activity compared with that of wild-type TRAIL or RGE-L-TRAIL (Fig. 5). Such enhanced activity could be attributed to $\alpha V\beta3$ and $\alpha V\beta5$ integrin ligand domain selectively bound to the integrin receptors, therefore, it enhanced the local concentration of TRAIL variant, to allow the TRAIL of the variant molecules to get access to TRAIL receptors more conveniently, frequently and effectively, and thus enhanced the signals to activate the cell apoptosis pathway. And such enhanced apoptosis activity depends on the integrin $\alpha V\beta3$ and $\alpha V\beta5$ abundance on the tumor cell surfaces. The higher the abundance, the higher the activity enhancement; for instance, among the MDA-MB-231 cells, due to the relatively low amount of integrin expression, its activity only enhanced for two times (Fig. 5). Not only so, for the COLO-205 colon cancer cells, when pre-incubation first with the competitive inhibition protein RGD-L-TRAIL (C230G) with intact ACDCRGDCFC domain and mutated TRAIL activity center, and then induced with RGD-L-TRAIL, the results revealed that the RGD-L-TRAIL-induced apoptosis activity declined significantly. The above results clearly indicated that the enhanced activity of TRAIL variant RGD-L-TRAIL was obtained by the targeting of ACDCRGDCFC. The fluorescein-labeled TRAIL tracking of its binding with the microvascular endothelial cells also demonstrated that TRAIL variant RGD-L-TRAIL has very high affinity with microvascular endothelial cells. However, the binding of the TRAIL with the fluorescein-labeled microvascular endothelial cells was very weak. The in vivo fluorescein-labeled and isotope-labeled test results further confirmed that: TRAIL variant could exist and specifically concentrated on the tumor tissues.

The ACDCRGDCFC domain of the TRAIL variant was mutated to ACDCRGECFC (RGE-L-TRAIL variant), while the RGE-L-TRAIL variant protein had lost the ability to bind with the integrins $\alpha V\beta5$ and $\alpha V\beta3$. The test results showed that there was nearly no difference in the activity between RGE-L-TRAIL and TRAIL, but much lower than that of RGD-L-TRAIL.

Different from the tumor cells, the normal human foreskin microvascular endothelial cells, 293T kidney cells and primary liver cells were treated with 300 ng/ml TRAIL variants or TRAIL for 24 hours; no obvious cell toxicity could be observed, which indicated that the TRAIL variants could distinguish the normal cells from tumor cells, could induce tumor cell apoptosis; but with relative safety for the normal cells.

The fluorescence detection method was used to test the activity of Caspase-8 and Caspase-3 in the Jurkat cells after treatment of TRAIL and TRAIL variants. After two hours of treatment, compared with the same dose of TRAIL, the TRAIL variant could induce higher Caspase-8 and Caspase-3 activity, 2.5 times as that of the wild-type TRAIL. Among the FADD$^{-/-}$ and Caspase-8$^{-/-}$ deficient Jurkat cells, no induced cell apoptosis could be tested for the TRAIL and TRAIL variants; which suggested that TRAIL variant played its induced apoptosis role through its death receptor --FADD--Caspase-8 pathway same as that of TRAIL.

Two kinds of colon cancer models COLO-205 and HT-29 were used to test the anti-tumor activity of TRAIL variant in the athymic nude mice. Because COLO-205 colon cancer cells were sensitive to TRAIL, the therapeutic effect of separate use of TRAIL and TRAIL variant was assessed on this model. On the 8th day after the nude mice were inoculated with tumor, administration of drugs through tail vein began, for ten days in succession, divided into four groups, respectively 100 $\mu$g TRAIL variant group, 20 $\mu$g TRAIL variant group, 100 $\mu$g wild-type TRAIL group and PBS control group. The statistical data showed that: compared with PBS control group, 100 $\mu$g of wild-type TRAIL would cause mild tumor shrinkage, and on the 37$^{th}$ day, the tumor inhibition rate was 37% (P = 0.08); however, in the 100 $\mu$g TRAIL variant group, the growth of tumor was significantly inhibited, particularly obvious during the treatment period, on the 19$^{th}$ day, the tumor inhibition rate was 70.4% (P = 0.002), and on the 37$^{th}$ day, the tumor inhibition rate was 56.8% (P = 0.016). Moreover, TRAIL variant's inhibition of tumor growth was dose-dependent. In the 20 $\mu$g TRAIL variant group, 37.1% (37 days, P = 0.045) tumor inhibition rate presented, slightly better than 5 times of dose (100 $\mu$g) wild-type TRAIL group.

The invention also provides the anti-tumor effect of combined use of TRAIL variant and chemotherapeutic drug CPT-11. Protein injection was carried out by intraperitoneal injection once a day for TRAIL or TRAIL variant, two week of injection in total, and intravenous injection once every two days from CPT-11 via tail veins, seven times in total. In the combined drug administration group, for the COLO-205 tumor models sensitive to TRAIL, a relatively low dose of CPT-11 (6.25 mg/kg/each) combined different doses of RGD-L-TRAIL (100 $\mu$g/day/each or 30 $\mu$g/day/each), or TRAIL (270 $\mu$g/day/each or 90 $\mu$g/day/each) was selected; Among the HT-29 colon cancer models resistant to TRAIL, relatively higher dose of CPT(25 mg/kg/each time) combined with RGD-L-TRAIL (200 $\mu$g/day/each) or TRAIL (400 $\mu$g/day/each) was selected. As shown in the Figures, among the COLO-205 tumor models, compared with PBS control groups, individual injection of low dose of CPT-11 could slightly inhibit the tumor growth; however, this dose of CPT-11 combined 100 µg RGD-L-TRAIL has significant effect of tumor inhibition (96.2%, P = 0.0001, 36 days), even has better effect than higher dose of TRAIL+CPT-11 combined treatment group (270 µg/day/each) (89%, P = 0.001, 36 days). Similarly, the tumor inhibition rate of 30 µg/day/each RGD-L-TRAIL group (77.1%, P=0.005, 36 days) was higher than the (90 µg/day/each dose of TRAIL group (67.8%, P = 0.012, 36 days). It is worth mentioning that, in the 100 µg/day/each dose of RGD-L-TRAIL+CPT-11 combined treatment group, after 36 days, among the 10 mice of drug administration, the tumors of 8 mice disappeared; and among ten animals of 270 µg/day/each TRAIL+CPT-11 combined treatment group, the tumors disappeared for 6 mice. The curative effect of the 100 µg/day/per dose of RGD-L-TRAIL + CPT-11 combined treatment group was better than that of the 270 µg/day/each TRAIL + CPT-11 combined treatment group.

Among the TRAIL-resistant HT-29 colon cancer models, when RGD-L-TRAIL was used separately, even when the daily intraperitoneal injection of 400 µg/each nude mouse, only mild anti-tumor effect was observed. However, when combined treatment with RGD-TRAIL + CPT-11, it could significantly inhibit the tumor growth, and presented better TRAIL+CPT-11 combined treatment effect. The group of 200 µg RGD-TRAIL plus 25 mg/kg CPT-11 combined treatment presented a better anti-tumor effect than that of 400 µg TRAIL and 25 mg/kg CPT-11 combined treatment group. This showed that, to achieve the same treatment effect, the dose required for TRAIL variant was far less than that of the wild-type TRAIL. Therefore, our results demonstrated that, the fusion of integrin αVβ3 and αVβ5 short peptide ligand ACDCRGDCFC peptide to TRAIL significantly enhanced its in vivo anti-tumor activity.

The in vivo animal model test further demonstrated that TRAIL variant has better curative effect than the wild-type TRAIL. Among the COLO-205 tumor models, the same dose of TRAIL variant has a significantly higher anti-tumor effect than that of the wild-type TRAIL, even when the dose of TRAIL variant is 1/5 of the dose of the wild-type TRAIL variant, its tumor inhibition rate is basically the same; which indicated that after fusion of TRAIL and tumor-targeting peptide, the in vivo level of anti-tumor biological activity indeed enhanced on the animal tumor models. Similarly, the drug efficacy of the TRAIL variant enhanced when used individually, when used combined with the chemotherapy drug CPT-11, it also has obvious synergistic effect, with more significant curative effect. Combined use of TRAIL variant and CPT-11 can not only reduce the dose and minimize the potential systemic toxicity, but also can expand to the treatment of tumors not sensitive to TRAIL. In the TRAIL-sensitive COLO-205 models, low dose of TRAIL variant (100 µg/day/each) combined with low dose of CPT-11 (6.25 mg/kg body weight) could extremely significant inhibit tumor growth and cause tumor recession, or even part of the tumor disappeared completely. To achieve the same therapeutic effect, if the combined treatment program is used, for the COLO-205 models, the dose required for the wild-type TRAIL is 3-9 times of that of TRAIL variant; for the TRAIL-insensitive HT-29 model, the dose required for the wild-type TRAIL is at least 2 times higher than that of the TRAIL variant. The in vivo animal test fully demonstrated that the therapeutic effect of TRAIL variant is better than that of TRAIL.

In order to confirm that the increased anti-tumor activity of TRAIL variant on animal models results from targeting concentration on the tumor tissues, we detected the distribution of I-125 isotope-labeled TRAIL variant RGD-TRAIL in the tumor tissues. The same radiation dose of I-125 labeled TRAIL and its variant were separately injected to COLO-205 tumor-bearing nude mice; 5, 30, 60, 120 and 240 minutes later, the mice were sacrificed to remove the tumor tissues and weigh them, then the isotope dose was measured in the liquid scintillation counter. The results showed that: after fusion of TRAIL with the integrin αVβ3 and αVβ5 short peptide ligand ACDCRGDCFC peptide, the targeting concentration of TRAIL proteins to COLO-205 tumor tissue regions enhanced obviously. During the initial injection period, the distribution of $^{125}$I-RGD-L-TRAIL in the tumor tissues was about twice as $^{125}$I-TRAIL. Due to the increased affinity of RGD-L-TRAIL on the tumor tissues and the increased distribution in tumor tissues, its clearing speed in the circulating blood significantly reduced, therefore, extending its distribution in the tumor tissues. 240 minutes later after injection, it is difficult to detect the TRAIL distribution in the tumor tissues, however, there were still many RGD-L-TRAIL continuous distribution in the tumor area. Therefore, the increased distribution of TRAIL variant in the tumor tissue areas and the enhanced anti-tumor activity on the animal models fully demonstrated that the TRAIL variant can endow the TRAIL with better tumor targeting, and could finally enhance the anti-tumor effect while reducing the drug dose.

**Claims**

1. A method of preparing a tumor necrosis factor related apoptosis inducing ligand (TRAIL) variant, being a fusion protein comprising an integrin ligand of αVβ3 and αVβ5, a tumor necrosis factor related apoptosis inducing ligand (TRAIL) and a linking peptide, wherein the linking peptide contains 2-20 amino acid residues, and wherein the integrin ligand is ACDCRGDCFC,

   wherein the method comprises the steps of constructing the coding genes for the TRAIL variant through artificial synthesis or gene cloning, and realizing the gene engineering expression and separation and purification through conventional genetic engineering,

   wherein E. coli recombinant strains of TRAIL variant with tumor tissue targeting are cultivated for 2.5 h at 37 °C, and then cultivated for 1 - 2 h at 24 °C, and then expression is induced by IPTG at 24 °C overnight, wherein the formation of inclusion body by the expression products is ultimately avoided effectively.

## Patentansprüche

1. Verfahren zur Herstellung einer Tumornekrosefaktor-verwandter Apoptose-induzierender Ligand (TRAIL)-Variante, wobei es sich um ein Fusionsprotein handelt, das einen Integrin-Liganden von αVβ3 und αVβ5, einen Tumornekrosefaktor-verwandter Apopto-se-induzierenden Liganden (TRAIL) und ein Verknüpfungspeptid umfasst, wobei das Verknüpfungspeptid 2 bis 20 Aminosäurereste enthält und wobei der Integrin-Ligand ACDCRGDCFC ist,
wobei das Verfahren die Schritte des Konstruierens der kodierenden Gene für die TRAIL-Variante durch künstliche Synthese oder Genklonierung und des Realisierens der gentechnischen Expression und Trennung und Reinigung mittels herkömmlicher Gentechnik umfasst,
wobei E. coli-rekombinante Stämme der TRAIL-Variante mit Tumorgewebezielsteuerung für 2,5 Stunden bei 37 °C kultiviert werden und dann für 1 bis 2 Stunden bei 24 °C kultiviert werden und dann eine Expression durch IPTG für 24 Stunden über Nacht induziert wird, wobei schließlich die Bildung eines Einschlusskörpers durch die Expressionsprodukte effektiv verhindert wird.

## Revendications

1. Procédé de préparation d'une variété de ligand (Tumor necrosis factor Related Apoptosis Inducing Ligand, TRAIL) inducteur d'apoptose lié au facteur de nécrose tumorale, étant une protéine de fusion comprenant un ligand d'intégrine de αVβ3 et αVβ5, une variété de ligand (TRAIL) inducteur d'apoptose lié au facteur de nécrose tumorale et un peptide de liaison, où le peptide de liaison contient 2 - 20 résidus d'acides aminés, et où le ligand d'intégrine est ACDCRGDCFC,
dans lequel le procédé comprend les étapes de construction des gènes codant pour le TRAIL pouvant être la synthèse artificielle ou le clonage de gènes, de réalisation de l'expression d'ingénierie génétique et de séparation et purification par des techniques conventionnelles d'ingénierie génétique,
dans lequel les souches recombinées d'E. Coli de TRAIL avec ciblage des tissus tumoraux sont cultivées pendant 2,5 h à 37 °C, puis cultivées pendant 1 - 2 h à 24 °C, puis l'expression est induite par IPTG à 24 °C pendant la nuit, où la formation du corps d'inclusion par le produit d'expression est finalement évitée efficacement.

1A

1B

**FIG. 1**

2A

2B

**FIG. 2**

**FIG. 3**

FIG. 4

FIG. 5

6A

6B

FIG. 6

FIG. 8

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6521228 B

**Non-patent literature cited in the description**

- **L. H. ZHAO et al.** *Letters in Biotechnology,* 2004, vol. 15 (3), 226-230

- **D. JACOB et al.** *Cancer Gene Therapy,* 2005, vol. 12 (2), 109-115